**Europäisches Patentamt**

**European Patent Office**

**Office européen des brevets**

(19)

(11) Veröffentlichungsnummer : **0 476 551 B1**

(12) **EUROPÄISCHE PATENTSCHRIFT**

(45) Veröffentlichungstag der Patentschrift :
21.12.94 Patentblatt 94/51

(51) Int. Cl.⁵ : **G02B 21/24, F16M 11/12**

(21) Anmeldenummer : **91115620.6**

(22) Anmeldetag : **14.09.91**

(54) **Tragevorrichtung mit Gewichtsausgleich für ein Operationsmikroskop.**

(30) Priorität : **19.09.90 DE 9013260 U**

(43) Veröffentlichungstag der Anmeldung :
**25.03.92 Patentblatt 92/13**

(45) Bekanntmachung des Hinweises auf die
Patenterteilung :
**21.12.94 Patentblatt 94/51**

(84) Benannte Vertragsstaaten :
**CH DE FR IT LI SE**

(56) Entgegenhaltungen :
**EP-A- 0 023 004**
**EP-A- 0 202 399**
**DE-C- 2 320 266**

(73) Patentinhaber : **Firma Carl Zeiss**
**D-73446 Oberkochen (DE)**

(72) Erfinder : **Heller, Rudolf**
**Köschenrüti-Strasse 12**
**CH-8052 Zürich (CH)**

**Beschreibung**

Die Erfindung betrifft eine Tragvorrichtung für ein räumlich verstellbares und in jeder Stellung fixierbares Anordnen eines Operationsmikroskopes nach dem Oberbegriff des Patentanspruchs.

Derartige Tragvorrichtungen sollen es einem Chirurgen ermöglichen, ein Operationsmikroskop nach Lage und Orientierung in einem vorbestimmten räumlichen Gebiet zu verstellen und zu fixieren, ohne daß dabei seine chirurgische Tätigkeit nachteilig beeinträchtigt wird. Sie sollen vor allem ohne einen den Chirurgen in seiner Tätigkeit belastenden Kraftaufwand verstellbar sein. Um die gewünschte Beweglichkeit des Operationsmikroskopes mit einem minimalen Kraftaufwand von Seiten des Chirurgen zu realisieren, ist es erforderlich, daß die Masse des zu bewegenden Operationsmikroskops durch ein Gegengewicht ausbalanciert wird.

Eine aus EP-A-0 023 004 bekannte Tragevorrichtung für ein Operationsmikroskop gemäß dem Oberbegriff des Patentanspruchs benützt eine in einem Gelenk des Zwischenträgerteils fixiert angebrachte Gegengewichtsscheibe und ein in Verlängerung der dem Hebel benachbarten ersten Parallelogrammseite angebrachtes Gegengewicht zum Ausbalancieren des Operationsmikroskops.

Bei einer anderen bekannten Tragevorrichtung dieser Art, die beispielsweise in der DE-PS 23 20 266 beschrieben ist, erfolgt die Ausbalancierung des Gewichtes des Operationsmikroskopes dadurch, daß an einem Zwischenträger und an einem Gestänge austauschbare und axial verstellbare Gewichte vorgesehen sind. Das Austauschen bzw. die Montage und Demontage von Gewichten ist jedoch bedienerunfreundlich und entspricht nicht den heutigen Ansprüchen an Operationsgeräte.

Die Erfindung hat es sich zur Aufgabe gemacht, diesen Mißstand zu beheben.

Sie löst diese Aufgabe durch die im kennzeichnenden Teil des Patentanspruchs angegebenen Merkmale.

Die mit der Erfindung erzielbaren Vorteile bestehen insbesondere darin, daß durch die stufenlose Verschiebbarkeit der Ausgleichgewichte eine optimale Ausbalancierung des Gewichts des Operationsmikroskopes möglich ist und in der bedienerfreundlichen Art der Massenkompensation, die ein An- und Abschrauben von Zusatzgewichten erübrigt.

Ein Ausführungsbeispiel der Erfindung ist in der Zeichnung dargestellt und wird im folgenden näher erläutert. Es zeigen

Fig. 1        eine schematische Darstellung der erfindungsgemäßen Tragvorrichtung in der Seitenansicht;

Fig. 2        die schematische Darstellung der Anordnung des Ausgleichgewichtes G1 in der Perspektive;

Fig. 3 und 4    eine prinzipielle Darstellung des Vorganges des Ausbalancierens des Gewichtes des Operationsmikroskopes.

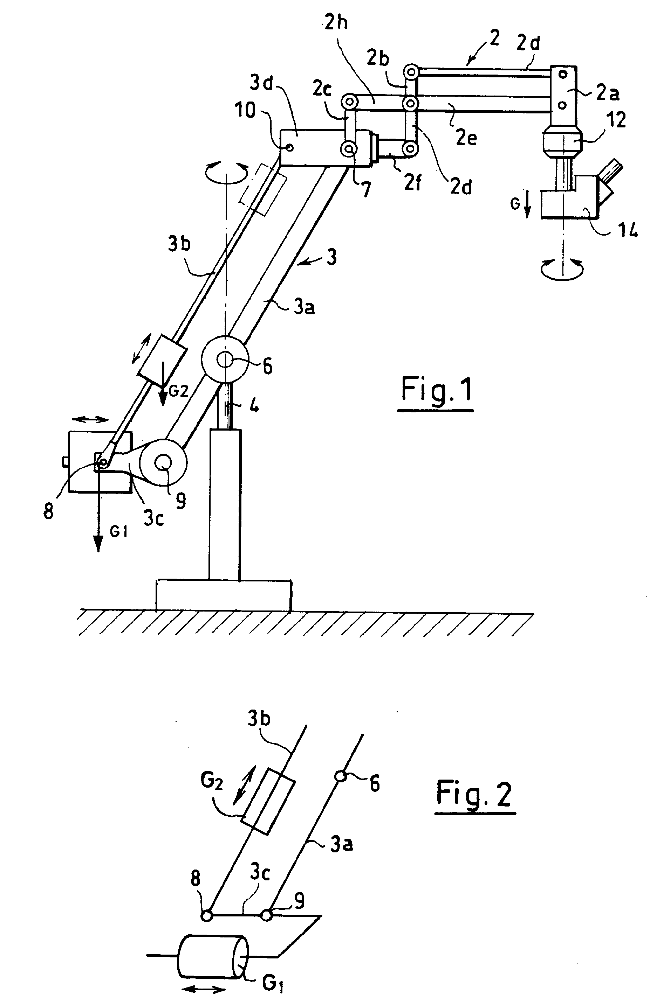

In der Figur 1 ist die in der Höhe verstellbare Trägersäule (4) der Tragvorrichtung mit (4) bezeichnet. Sie ist über ein Drehlager (6) mit einem Gelenkparallelogramm (3) verbunden. Eine Seite des Gelenkparallelogramms (3) ist als zweiarmiger Hebel (3a) ausgeführt, der im Drehlager (6) drehbar ist. Die Gegenseite zum Hebel (3a) ist als Stange (3b) ausgeführt, auf der ein Gewicht G2 in der angedeuteten Pfeilrichtung axial verschiebbar angeordnet ist. Im Verbindungsgelenk (9) des Hebels (3a) ist parallel zur Parallelogrammseite (3c) ein weiteres in der dort angedeuteten Pfeilrichtung verschiebbares Ausgleichsgewicht (G1) angeordnet. Das Verbindungsgelenk (7) des Hebels (3a) mit seiner benachbarten Parallelogrammseite (3d) ist mit dem Doppelgelenkparallelogramm (2) verbunden, das über ein Drehlager (12) das Operationsmikroskop (14) trägt.

In der in Fig. 2 schematisch-perspektivisch dargestellten Halterung des Ausgleichsgewichts G1 ist das Gestänge zur Halterung des Gewichts mit dem Bezugszeichen (13) gekennzeichnet. Das Gewicht ist in der angedeuteten Pfeilrichtung verschiebbar. Die schematisch gezeichneten Seiten und Gelenke des Gelenkparallelogramms (3) tragen dieselbe Bezeichnung wie in der Darstellung der Figur 1.

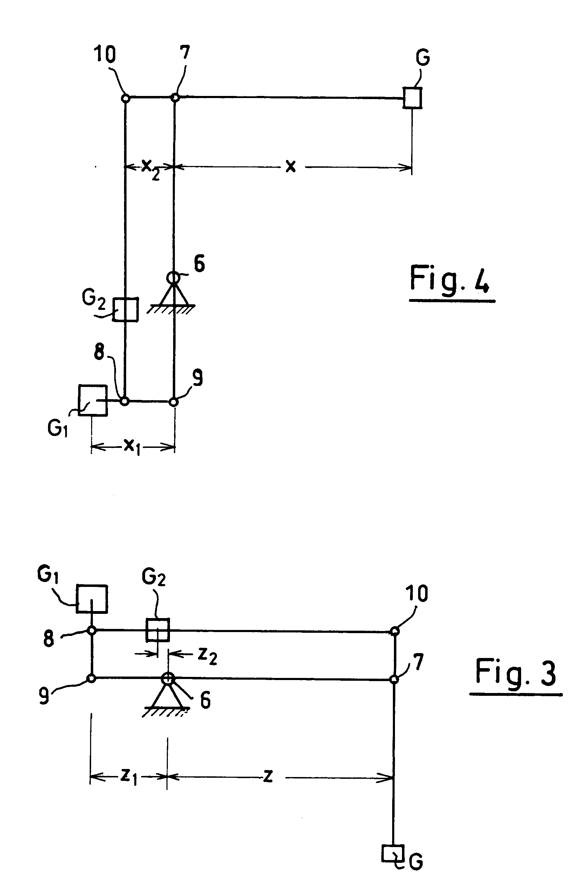

In den Figuren 3 und 4 ist die schrittweise Ausbalancierung des Gewichts G des Operationsmikroskops (14) durch die Gegengewichte G1 und G2 in zwei Stellungen der Tragvorrichtung dargestellt. Zuerst wird das Gelenkparallelogramm (3) wie in Figur 3 gezeigt, nach vorne gekippt, d.h. in Horizontallage gebracht. Die Gleichgewichtsbedingung für diese Lage lautet:

$$G1 \cdot Z1 + G2 \cdot Z2 = G \cdot Z$$

Da in dieser Gleichung die Werte G1, Z1, G2 und Z konstant sind, besteht ein formaler Zusammenhang zwischen dem Mikroskopgewicht G und dem Hebelarm Z2 des Gegengewichts G2. Der Hebelarm (Z2) läßt sich zur Ausbalanzierung stufenlos einstellen.

Der zweite, in Fig. 4 dargestellte Schritt, erfolgt an der wieder aufgerichteten Tragvorrichtung. Diese Stellung weist folgende Gleichgewichtsbedingungen auf:

$$G1 \cdot X1 + G2 \cdot X2 = G \cdot X$$

Die Werte dieser Gleichung sind mit Ausnahme von X1 und G unveränderlich. Zur Ausbalancierung wird der Hebelarm X1 des Ausgleichsgewichts G1 eingestellt.

**Patentansprüche**

1. Tragvorrichtung für ein räumlich verstellbares und in jeder Stellung fixierbares Operationsmikroskop (14), bestehend aus einem als Doppelgelenkparallelogramm (2) ausgeführten Geräteträgerteil, an dem einerseits die Montage des Operationsmikroskops vorgesehen ist, und das andererseits mit einem Zwischenträgerteil (3) verbunden ist, welches seinerseits mit einer vertikalen Trägersäule (4) verbunden und relativ zu dieser verstellbar ist, und welches als Gelenkparallelogramm (3) ausgeführt ist, von dem eine Seite als zweiarmiger Hebel (3a) ausgeführt ist, der in einem mit der Trägersäule (4) verbundenem Drehlager (6) drehbar ist, und bei welchem am Verbindungsgelenk (9) des Hebels (3a) mit einer ersten benachbarten Parallelogrammseite (3c) ein Gestänge (13) für ein erstes Ausgleichsgewicht (G1) angeordnet ist, während das Verbindungsgelenk (7) des Hebels (3a) mit seiner zweiten benachbarten Parallelogrammseite (3d) mit dem als Doppelgelenkparallelogramm ausgeführten Geräteträgerteil (2) verbunden ist, dadurch gekennzeichnet, daß die dem Hebel (3a) gegenüberliegende Parallelogrammseite als Stange (3b) ausgeführt ist, auf der ein zweites Ausgleichsgewicht (G2) axial verschiebbar angeordnet ist, und daß das Gestänge so angeordnet ist, daß das erste Ausgleichsgewicht (G1) parallel zu der ersten dem Hebel (3a) benachbarten Parallelogrammseite (3c) verschiebbar ist.

**Claims**

1. Supporting frame for a spatially-adjustable surgical microscope (14) which can be locked in any adjusted position, comprising an instrument carrying portion being configured as a coupled-hinge parallelogram (2) for mounting said surgical microscope (14);
said coupled-hinge parallelogram (2) being connected with an intermediate support portion (3);
said intermediate support portion (3) being connected with a vertical support column (4) and being relatively movable with respect to said vertical support column (4);
said intermediate support portion (3) being configured as a hinged parallelogram (3);
one side of said hinged parallelogram (3) being a two-armed lever (3a) which is rotatable about a pivot bearing (6) that is connected with said vertical support column (4);
said two-armed lever (3a) having a connecting hinge (9) which connects the lever (3a) with the adjacent first side (3c) of the hinged parallelogramm and said conecting hinge (9) having a rod assembly (13) for carrying a first counterweight (G1);
said two-armed lever (3a) having a connecting hinge (7) which connects the lever (3a) with the adjacent second side (3d) of said hinged parallelogramm (3) with said instrument carrying portion being configured as a coupled-hinge parallelogram (2),
**characterized by the fact, that**
the side of the hinged parallelogram (3) which is located opposite to the two-armed lever (3a) being configured as a rod (3b) with an axially movable counterweight (G2) and said rod assembly (13) being configured so that the first counterweight (G1) being movable parallel to said first adjacent side (3c) of the hinged parallelogramm (3).

**Revendications**

1. Dispositif de support pour un microscope chirurgical (14) réglable dans l'espace et pouvant être fixé dans n'importe quelle position, comprenant une partie de support d'appareils réalisée selon un parallélogramme (2) à double articulation sur laquelle, d'une part, est prévu le montage du microscope chirurgical et qui, d'autre part, est reliée à une partie de support intermédiaire (3), laquelle est reliée de son côté à une colonne de support (4) verticale et est réglable par rapport à celle-ci, et qui est réalisée selon un parallélogramme articulé (3), dont un côté est réalisé comme un levier (3a) à deux bras qui peut tourner dans un coussinet de pivotement (6) relié à la colonne de support (4), le côté du parallélogramme faisant face au levier (3a) est réalisé comme une tige (3b) sur laquelle un deuxième poids d'équilibrage (G2) est disposé de manière à pouvoir se déplacer axialement et pour lequel, à l'articulation de liaison (9) du levier (3a), des tiges (13) sont agencées pour un premier poids d'équilibrage (G1) avec un premier côté (3c) de parallélogramme adjacent, tandis que l'articulation de liaison (7) du levier (3a) est reliée avec son deuxième côté (3d) de parallélogramme adjacent à la partie de support d'appareils (2) réalisée comme un parallélogramme à double articulation, caractérisé en ce que le côté du parallélogramme faisant face au levier (3a) est réalisé comme une tige (3b) sur laquelle un deuxième poids d'équilibrage (G2) est disposé de manière à pouvoir se déplacer axialement et en ce que les tiges sont agencées de sorte que le premier poids d'équilibrage G1 parallèle au premier côté du parallélogramme (3c) adjacent au levier (3a) peut être déplacé.

Fig.1

Fig.2

EP 0 476 551 B1

Fig. 4

Fig. 3

6